# EUROPEAN PATENT SPECIFICATION

(11) **EP 3 782 701 B1**
(45) Date of publication and mention of the grant of the patent: **07.06.2023**
(21) Application number: 18914419.9
(22) Date of filing: 02.07.2018
(51) Int. Cl.: A61N 5/06

(54) **IMMUNE-INDUCTION THERAPEUTIC APPARATUS**
IMMUNINDUKTIONSTHERAPIEVORRICHTUNG
APPAREIL THÉRAPEUTIQUE À INDUCTION IMMUNITAIRE

(30) Priority: 14.04.2018 CN 201810334267
(43) Date of publication of application: 24.02.2021
(73) Proprietor: Beijing Yanyang Med-Tech Company Limited, Beijing 100032 (CN); Liaoning Yanyang Medical Instrument Co., Ltd, Shenyang, Liaoning 110000 (CN)
(72) Inventor: FENG, Bo, Shenyang, Liaoning 110000 (CN); GAO, Xinghua, Shenyang, Liaoning 110001 (CN); QI, Ruiqun, Shenyang, Liaoning 110001 (CN); CAO, Tiejun, Beijing 100032 (CN)
(74) Representative: ZHAOffice SPRL
(86) International application number: PCT/CN2018/093969
(87) International publication number: WO 2019/196212

(56) References cited:
- WO-A1-2014/044125
- CN-A- 102 526 893
- CN-A- 108 421 166
- CN-B- 102 526 893
- CN-U- 204 485 103
- CN-U- 204 485 103
- CN-U- 205 698 923

## Description

### Technical Field

The present invention belongs to the technical field of therapy apparatus, and in particular, relates to an immunity induction therapy apparatus.

### Background

From the perspective of clinical medicine, a variety of physical methods can be used to treat skin diseases. Commonly used are laser, cryotherapy, thermotherapy, electrocauterization, etc. In the treatment of some tumorous and neoplastic skin diseases, appropriate physical methods are often used for destructive removal, such as laser cauterization for condyloma acuminatum, and liquid nitrogen cryotherapy for Bowen's disease (precancerous lesion). These treatments may all cause some trauma, which might be unbearable and bring great pain to patients. In addition, because the skin has the functions of health protection and beauty, etc., it is necessary to give consideration to both curing diseases and avoiding disfigurement during the treatment of skin diseases. Therefore, some destructive methods to treat some skin tumors and neoplasms have great disadvantages.

International Patent Application WO2014/044125 discloses a laser and infrared-photon magnetic constipation instrument comprises a controller assembly, an output end assembly, a protecting band , and a cable. the output end assembly comprises a front case, a rear case, and a middle case, a laser assembly, an infrared source, and a magnet being provided inside the case body of the output end assembly. The laser assembly comprising a laser source and a laser rack; a thermistor is provided in the output end assembly, and the infrared source, the laser source, a laser switch, and the thermistor are connected to the PCB assembly through the cable. The disclosed instrument uses the laser, infrared light and magnetic force to output mode simultaneously, and the therapy simulates the traditional Chinese medicine acupuncture therapy and modern magnetic therapy. This document s considered the closest prior art.Chinese utility model 204 485 103 discloses a laser treatment device for bright red spots.

The device comprises a laser generating module, a display device, a treatment head assembly and a housing. The display device is used to control the laser The generating module outputs the laser with a set power, and the laser is guided to the patient's bright red spot for treatment through the treatment head assembly.

### Summary of the Invention

The present invention provides a nondestructive immunity induction therapy apparatus to solve the aforementioned problems.

In order to achieve the aforementioned objective, the present invention uses the following technical solution: the present invention includes a infrared light generating device, and a bearing and driving part configured to bear the infrared light generating device and capable of driving the infrared light generating device to be disposed at a suitable position. The immunity induction therapy apparatus is structurally wherein the infrared light generating device includes a light source part and an optical system configured to regulate and output light emitted by the light source part, and the light source part is connected to a light source control circuit.

In the present invention, the optical system comprises a light source focusing group, a light homogenizing group, a homogenized-light focusing and focus regulating group, and a homogenized-light collimating group arranged in sequence from front to back; the light source part comprises a reflector cup and a light source provided at the center of the reflector cup; and the reflector cup is provided on the front side of the light source focusing group and the light homogenizing group uses an integrator rod.

As another preferred solution, in the present invention, the light source focusing group uses two convex lenses, or uses one convex lens, or uses two plano-convex lenses and one biconvex lens, or uses a nonstandard spherical lens.

As another preferred solution, in the present invention, the integrator rod has a diameter of 7 mm and a length of 40 mm.

As another preferred solution, in the present invention, the integrator rod is a cylindrical glass rod with both ends coated with antireflection films and an outer side wall coated with an internal reflection film.

As another preferred solution, in the present invention, A front convex lens in the two convex lenses of the light source focusing group has a front surface radius of 18 mm, a second surface radius of -17 mm, and a center thickness of 4.95 mm; and a rear convex lens in the two convex lenses of the light source focusing group has a front surface radius of 24 mm, a second surface radius of -24 mm, and a center thickness of 3.25 mm.

As another preferred solution, in the present invention, the homogenized-light focusing and focus regulating group uses two convex lenses, or uses one convex lens.

As another preferred solution, in the present invention, a front convex lens in the two convex lenses of the homogenized-light focusing and focus regulating group has a front surface radius of 150.96 mm, a second surface radius of -4.17 mm, and a center thickness of 4.98 mm; and a rear convex lens in the two convex lenses of homogenized-light focusing and focus regulating group has a front surface radius of 150.96 mm, a second surface radius of -4.17 mm, and a center thickness of 4.98 mm.

As another preferred solution, in the present invention, the homogenized-light collimating group uses a lens, and the lens has a rear surface radius of 17.5 mm.

As another preferred solution, in the present invention, the distance from a rear end of the reflector cup to the front convex lens of the light source focusing group is 41.14 mm, the distance between the front convex lens of the light source focusing group and the rear convex lens of the light source focusing group is 0.25 mm, the distance from the rear convex lens of the light source focusing group to the integrator rod is 4.29 mm, the distance from the light homogenizing group to the front convex lens of the homogenized-light focusing and focus regulating group is 0.47mm, the distance between the front convex lens of the homogenized-light focusing and focus regulating group and the rear convex lens of the homogenized-light focusing and focus regulating group is 4 mm, and the distance between the rear convex lens of the homogenized-light focusing and focus regulating group and the homogenized-light collimating group is 45.29 mm.

As another preferred solution, in the present invention, the reflector cup uses a nonlinear secondary reflector cup.

As another preferred solution, in the present invention, the light source uses a halogen tungsten lamp.

As another preferred solution, in the present invention, the infrared light generating device comprises a treatment head housing, and a front end of the treatment head housing is a light outlet hole; a light extinction barrel mounting base, a rear light extinction barrel, a front light extinction barrel, and an optical tube are arranged in the treatment head housing in sequence from back to front; and the light source part is provided in the rear of the rear light extinction barrel, and the optical system is provided in the optical tube.

As another preferred solution, in the present invention, the bearing and driving part comprises a base, a carrier body, and a rocker arm assembly; the carrier body is provided on the base; one end of the rocker arm assembly is connected to an upper end of the carrier body, and the other end of the rocker arm assembly is connected to the treatment head housing; and the light source control circuit is provided in the carrier body.

As another preferred solution, in the present invention, the light source control circuit comprises a main control part, a lesion temperature detection part, a power supply part, and an output regulation part; a detection signal input port of the main control part is connected to a detection signal output port of the lesion temperature detection part, and a control signal output port of the main control part is connected to a control signal input port of the output regulation part; and an electric energy output port of the power supply part is connected to an electric energy input port of the main control part, an electric energy input port of the lesion temperature detection part, and an electric energy input port of the output regulation part, respectively.

As another preferred solution, the present invention further comprises a treatment head temperature detection part and a carrier body temperature detection part; and the detection signal input port of the main control part is connected to a detection signal output port of the treatment head temperature detection part and a detection signal output port of the carrier body temperature detection part, respectively.

As another preferred solution, the present invention further comprises a system operation state indication part, a system alarm state indication part, and a human-computer interaction part; the control signal output port of the main control part is connected to a control signal input port of the system operation state indication part and a control signal input port of the system alarm state indication part, respectively; a signal transmission port of the main control part is connected to a signal transmission port of the human-computer interaction part; and an electric energy input port of the human-computer interaction part is connected to the electric energy output port of the power supply part.

As another preferred solution, the present invention further comprises a treatment head heat dissipation part and a carrier body heat dissipation part; an electric energy input port of the treatment head heat dissipation part and an electric energy input port of the carrier body heat dissipation part are respectively connected to the electric energy output port of the power supply part.

As another preferred solution, in the present invention, the output regulation part uses a solid-state voltage regulator.

As another preferred solution, in the present invention, the lesion temperature detection part uses an infrared temperature sensor.

As another preferred solution, in the present invention, an alarm is activated when the temperature detected by the treatment head temperature detection part is higher than 80 degrees Celsius, and the alarm is canceled when the temperature is lower than 40 degrees Celsius;
an alarm is activated when the temperature detected by the carrier body temperature detection part is higher than 50 degrees Celsius, and the alarm is canceled when the temperature is lower than 40 degrees Celsius; and
an alarm is activated when the temperature detected by the lesion temperature detection part is higher than 50 degrees Celsius, and the alarm is canceled when the temperature is lower than 50 degrees Celsius.

As another preferred solution, in the present invention, the power supply part comprises a pulse train suppressor, a first filter, a second filter, and a direct-current power source; an input port of the pulse train suppressor is a mains access port; an output port of the pulse train suppressor is connected to an input port of the second filter, an input port of the direct-current power source, and the electric energy input port of the carrier body heat dissipation part, respectively, through the first filter; an output port of the second filter is connected to the electric energy input port of the output regulation part; and an output port of the direct-current power source is connected to the electric energy input port of the main control part, the electric energy input port of the lesion temperature detection part, the electric energy input port of the human-computer interaction part, and the electric energy input port of the treatment head heat dissipation part, respectively.

As another preferred solution, in the present invention, the pulse train suppressor uses a DTS4221-3NE-W20-D16 pulse train suppressor A16; the first filter and the second filter use RSHN-2006L filters A1 and A2; and the direct-current power source uses an MSP-100-24 direct-current power source A3.

An input L port of A16 is connected to a wiring terminal of an SB2 output end of a GQ22-11ZE220V switch; the other wiring terminal of the SB2 output end is respectively connected to pin 2 of A2, pin 3 of A1, an N port of A3, and an electric energy input N port of the carrier body heat dissipation part; an SB2 input end is a mains L wiring terminal; an input N port of A16 is a mains N wiring terminal; and an input PE port of A16 is a mains PE wiring terminal.

An output L port of A16 is connected to pin 1 of A1 through a switch SB1 and a ceramic fuse FU1 in sequence; an output N port of A16 is connected to pin 2 of A1; pin 4 of A1 is connected to pin 1 of A2, one end of a ceramic fuse FU2, and an electric energy input L port of the carrier body heat dissipation part, respectively; and the other end of the ceramic fuse FU2 is connected to port L of A3.

As another preferred solution, in the present invention, the main control part comprises a PLC controller, an analog output module, and an analog input module; a detection signal input port of the PLC controller is connected to the detection signal output port of the lesion temperature detection part, and a signal transmission port of the PLC controller is connected to the signal transmission port of the human-computer interaction part; the analog output module is connected to the control signal input port of the output regulation part and the control signal input port of the system alarm state indication part, respectively; and the analog input module is connected to the detection signal output port of the treatment head temperature detection part and the detection signal output port of the carrier body temperature detection part, respectively.

As another preferred solution, in the present invention, the PLC controller uses a 6ES7214-1HG40-0XB0 controller A6; the analog output module uses a 6ES7232-4HB32-0XB0 module A7; the analog input module uses a 6ES7231-5PD32-0XB0 module A8; port L+ of A6, port L+ of A7, and port L+ of A8 are all connected to port +V of the MSP-100-24 direct-current power source A3; and port M of A6, port M of A7, and port M of A8 are all connected to port -V of A3.Port 0 of a DI part of A6 is connected to port +V of A3 through a GQ16F-10 silencer button SB3, port 0M of the DI part of A6 is connected to the port -V of A3, and port 2M of an AI part of A6 is connected to the port -V of A3.Port 1L of a DQa part of A6 is connected to the port +V of A3.

As another preferred solution, in the present invention, the carrier body heat dissipation part uses a carrier body interior heat dissipation fan, and the treatment head heat dissipation part uses a treatment head interior heat dissipation fan.

As another preferred solution, in the present invention, the infrared temperature sensor uses an MLX90614-ESF-BCF sensor A17; pin 1 of A17 is connected to port 0 of the AI part of A6, pin 2 of A17 is connected to port -V of A3, and pins 3 and 4 of A17 are connected to the port +V of A3.

As another preferred solution, in the present invention, the human-computer interaction part uses a touch screen.

As another preferred solution, in the present invention, the touch screen uses a 6AV2124-0GC01-0AX0 touch screen A9; port L+ of A9 is connected to the port +V of A3, port M of A9 is connected to the port -V of A3, and port X1P1 of A9 is connected to port X1P1 of A6.

As another preferred solution, in the present invention, the system operation state indication part uses an indicator lamp HL1; the system alarm state indication part comprises an indicator lamp HL2 and a buzzer A18; port 0 of a DQa part of A6 is connected to the port -V of A3 through HL1, and port 1 of the DQa part of A6 is connected to the port -V of A3 through HL2; a control signal input port of A18 is connected to port 1M and port 1 of A7, and a power port of A18 is connected to the ports +V and -V of A3, respectively.

As another preferred solution, in the present invention, the solid-state voltage regulator uses an LTVDH-220V-15A solid-state voltage regulator A4; pin 1 of A4 is connected to one end of a TC primary side of a 220VAC/15VAC/200W transformer through a ceramic fuse FU3; the other end of the TC primary side is connected to pin 3 of A2 and pin 4 of A4, respectively; pin 4 of A2 is connected to pins 2 and 3 of A4, respectively; one end of a TC secondary side is connected to one end of a power terminal of the light source through a ceramic fuse FU4; the other end of the power terminal of the light source is connected to the other end of the TC secondary side; and pin 7 of A4 is connected to port 0M of A7, and pin 6 of A4 is connected to port 0 of A7.

As another preferred solution, in the present invention, the treatment head temperature detection part comprises two thermistors; one end of one of the thermistors is connected to port M+ and port I+ of an AI0 part of A8, respectively, and the other end is connected to port M- and port I- of the AI0 part of A8, respectively; one end of the other thermistor is connected to port M+ and port I+ of an AI1 part of A8, respectively, and the other end is connected to port M- and port I- of the AI1 part of A8, respectively.

As another preferred solution, in the present invention, the carrier body temperature detection part comprises two thermistors; one end of one of the thermistors is connected to port M+ and port I+ of an AI2 part of A8, respectively, and the other end is connected to port M- and port I- of the AI2 part of A8, respectively; one end of the other thermistor is connected to port M+ and port I+ of an AI3 part of A8, respectively, and the other end is connected to port M- and port I- of the AI3 part of A8, respectively.

As another preferred solution, in the present invention, the treatment head housing comprises a shell with portions on two sides snap-fitted together and a rear end protective cover; the protective cover is threadedly connected to the shell; and the protective cover is provided with strip-shaped heat dissipation holes.

As another preferred solution, in the present invention, the rear end of the light extinction barrel mounting base is provided with the treatment head interior heat dissipation fan.

As another preferred solution, in the present invention, the rear light extinction barrel and the front light extinction barrel both use aluminum alloy light extinction barrels, and the inner walls thereof are subjected to extinction processing; the front end of the front light extinction barrel is provided with an internal threaded hole connected to the rear end of the optical tube, and the front light extinction barrel around the internal thread hole is provided with arc-shaped heat dissipation holes; the reflector cup mounting base is provided in the rear light extinction barrel, and the reflector cup mounting base comprises a mounting hole in the rear end and support arms on two sides; the front ends of the support arms are bent inward, and the end faces are in an arc shape corresponding to the front arc-shaped surface of the reflector cup.

As another preferred solution, in the present invention, the optical tube comprises a front half and a rear half; the front half is threadedly connected to the rear half; the rear end of the rear half is provided with an external thread connected to the front light extinction barrel; both the rear end of the rear half and the front end of the rear half are provided with lens fixing members; the lens fixing members are threadedly connected to the optical tube; and the middle of the optical tube is provided with a light homogenizing group mounting member.

As another preferred solution, in the present invention, the shell uses an ABS engineering plastic shell, and the front side wall of the shell is provided with strip-shaped heat dissipation holes.

As another preferred solution, in the present invention, a infrared light generating device, the total duration of a single light exposure is 1,740 seconds, heating is completed to present temperature within 60 seconds, maintaining the preset temperature is adjusted in 61-240 seconds, the cooling is in 241-420 seconds, reduced to less than the preset temperature 1.5 degrees Celsius and adjusted, at the same time satisfied tan45 degrees (Angle of temperature rise curve), the heating is in 421-600 seconds, increased to more than the preset temperature 1.5 degree Celsius and adjusted, at the same time satisfied tan45 degrees, the cooling is in 601-660 seconds, reduced to the preset temperature, the cooling is in 661-840 seconds, reduced to less than the preset temperature 1.8 degree Celsius and adjusted, at the same time satisfied tan45 degrees, the heating is in 841-1020 seconds, increased to more than preset temperature 1.8 degree Celsius and adjusted, at the same time satisfied tan45 degrees, the cooling is in 1021-1080 seconds, reduced to preset temperature, the cooling is in 1081-1200 seconds, reduced to less than preset temperature 1.8 degree Celsius and adjusted, at the same time satisfied tan45 degrees, the heating is in 1021-1320 seconds, increased to more than preset temperature 1.8 degrees Celsius and adjusted, at the same time satisfied tan45 degrees, the cooling is in 1321-1380 seconds, reduced to less than preset temperature, the cooling is in 1381-1500 seconds, reduced to less than the preset temperature 1.8 degree Celsius and adjusted, at the same time satisfied tan45 degrees, the heating is in 1501-1620 seconds, increased to more than preset temperature 1.8 degrees Celsius and adjusted, at the same time satisfied tan45 degrees, the cooling is in 1621-1740 seconds, reduced to preset temperature.

As another preferred solution, in the present invention, the preset temperature is 41, 42, 43 or 44°C.

As another preferred solution, in the present invention, when the preset temperature is maintained in 61-240 seconds: the peak value of infrared light wave with 60% of the maintenance power in 61-75 seconds is 0.8-1.2um, and the preset temperature is adjusted in 76-240 seconds.

As another preferred solution, in the present invention, the heating is in 421-600 seconds: the peak value of infrared light wave with 54% of the maintenance power in 421-435 seconds is 1.5-1.7um, and the heating is in 436-600 seconds.

As another preferred solution, in the present invention, the cooling is in 601-660 seconds: the peak value of infrared light wave with 38% of the maintenance power in 601-615 seconds is 2.5-2.7um, and the cooling is in 616-660 seconds.

As another preferred solution, in the present invention, the cooling is in 661-840 seconds: the peak value of infrared light wave with 42% of maintenance power in 661-675 seconds is 2.2-2.6um, and the cooling is in 676-840 seconds.

As another preferred solution, in the present invention, the heating is in 841-1020 seconds: the peak value of infrared light wave with 30% of maintenance power in 841-855 seconds is 2.8-3.0um, and the heating is in 856-1020 seconds.

As another preferred solution, in the present invention, the cooling is in 1021-1080 seconds: the peak value of infrared light wave with 56% of maintenance power in 1021-1035 seconds is 1.7-1.9um, and the cooling is in 1036-1080 seconds.

As another preferred solution, in the present invention, the cooling is in 1081-1200 seconds: the peak value of infrared light wave with 60% of maintenance power in 1081-1095 seconds is 0.8-1.2um, and the cooling is in 1096-1200 seconds.

As another preferred solution, in the present invention, the heating is in 1021-1320 seconds: the peak value of infrared light wave with 40% of maintenance power in 1021-1035 seconds is 2.6-2.8um, and the cooling is in 1036-1320 seconds.

As another preferred solution, in the present invention, the cooling is in 1321-1380 seconds: the peak value of infrared light wave with 47% of maintenance power in 1321-1335 seconds is 1.8-2.1um, and the cooling is in 1336-1380 seconds.

As another preferred solution, in the present invention, the cooling is in 1381-1500 seconds: the peak value of infrared light wave with 60% of maintenance power in 1381-1395 seconds is 0.8-1.2um and the cooling is in 1395-1500 seconds.

As another preferred solution, in the present invention, the heating is in 1501-1620 seconds: the peak value of infrared light wave with 30% of maintenance power in 1501-1515 seconds is 2.8-3.0um and the heating is in 1516-1620 seconds.

As another preferred solution, in the present invention, the cooling is in 1621-1740 seconds: the peak value of infrared light wave with 54% of maintenance power in 1621-1635 seconds is 1.5-1.7um and the cooling is down to present temperature in 1636-1740 seconds

As another preferred solution, in the present invention, the present temperature is 41, 42, 43 or 44°C.

As another preferred solution, in the present invention, the front end of the treatment head housing is provided with a limiting component, the limiting component has a limiting silicone head protruding forward, and the lesion temperature detection part is provided on the limiting component.

As another preferred solution, in the present invention, the treatment head temperature detection part is provided in the treatment head housing.

As another preferred solution, in the present invention, the base comprises a base plate; a base plate cover is snapped onto the base plate; the middle of the base plate cover is provided with a carrier body mounting groove, and the lower ends of the four corners of the base plate are provided with wheels.

As another preferred solution, in the present invention, the wheels use 10-inch universal wheels.

As another preferred solution, in the present invention, the base plate uses an iron base plate, the upper ends of the four corners of the base plate are welded with U-shaped reinforcement profiles, and the base plate is provided with carrier body connecting holes.

As another preferred solution, in the present invention, the base plate cover uses an ABS engineering plastic base plate cover.

As another preferred solution, in the present invention, the carrier body comprises a structural frame; the carrier body housing is provided around the structural frame; the upper end of the carrier body housing is provided with an armrest and the human-computer interaction part; the lower end of the structural frame is connected to the base plate; and the light source control circuit is provided in the structural frame.

As another preferred solution, in the present invention, the armrest is a U-shaped armrest surrounding two lateral sides and the rear of the carrier body, and the rear end of the armrest is tilted upward.

As another preferred solution, in the present invention, the front carrier body housing of the carrier body is an L-shaped structure; a vertical surface of the front end of the front carrier body housing is provided with a vertical treatment head receiving groove, and a horizontal surface of the rear upper end of the front carrier body housing is connected to the front upper end of the structural frame; both sides of the front end of the armrest are bent inward, and the bend of the front carrier body housing is provided with connecting insertion holes corresponding to the bent portions on the front end of the armrest.

As another preferred solution, in the present invention, the carrier body housing is connected to the structural frame through an inserting structure and screws.

As another preferred solution, in the present invention, the carrier body housing uses an ABS engineering plastic housing.

As another preferred solution, in the present invention, the structural frame uses a metal structural frame.

As another preferred solution, in the present invention, the rocker arm assembly comprises an upright column, a horizontal rocker arm, a connecting rocker arm, and a handle; a lower end of the upright column is connected to the carrier body, and an upper end of the upright column is connected to a rear end of the horizontal rocker arm through a first vertical rotating shaft; a front end of the horizontal rocker arm is connected to a rear end of a horizontal connecting block through a second vertical rotating shaft; a front end of the horizontal connecting block is connected to a rear end of the connecting rocker arm through a first horizontal rotating shaft; a front end of the connecting rocker arm is connected to an upper end of a vertical connecting block through a second horizontal rotating shaft; a lower end of the vertical connecting block is connected to an upper end of the handle through a third vertical rotating shaft; and a lower end of the handle is connected to the treatment head housing.

As another preferred solution, in the present invention, the lower end of the handle is connected to the treatment head housing through a wire-passing damping shaft.

Further, in the present invention, the handle is a horizontal double U-shaped handle; upper ends of U-shaped handles on two sides are bent toward each other and connected to the lower end of the vertical connecting block through the third vertical rotating shaft, and lower ends of the U-shaped handles on both sides are bent toward each other and connected to both sides of the treatment head housing.

In addition, in the present invention, the lower ends of the U-shaped handles on both sides are bent toward each other and connected to both sides of the treatment head housing through the wire-passing damping shaft.

The present invention has the following beneficial effects.

The present invention uses the infrared light generating device to perform immune induction treatment on skin lesions without causing trauma to a human body, thus being more bearable and causing no pain for patients, and therefore provides a non-destructive treatment device.

The bearing and driving part of the present invention can dispose the infrared light generating device at a suitable position, thereby facilitating the treatment of human lesions.

The present invention improves the effect of light therapy by regulating the light emitted by the light source part by means of the optical system.

The present invention is provided with a light source control circuit, thereby facilitating the relevant control of the light source.

### Brief Description of the Drawings

The present invention is further described below with reference to the accompanying drawings and the specific embodiments. The scope of protection of the present invention is not limited to the expression of the following content.

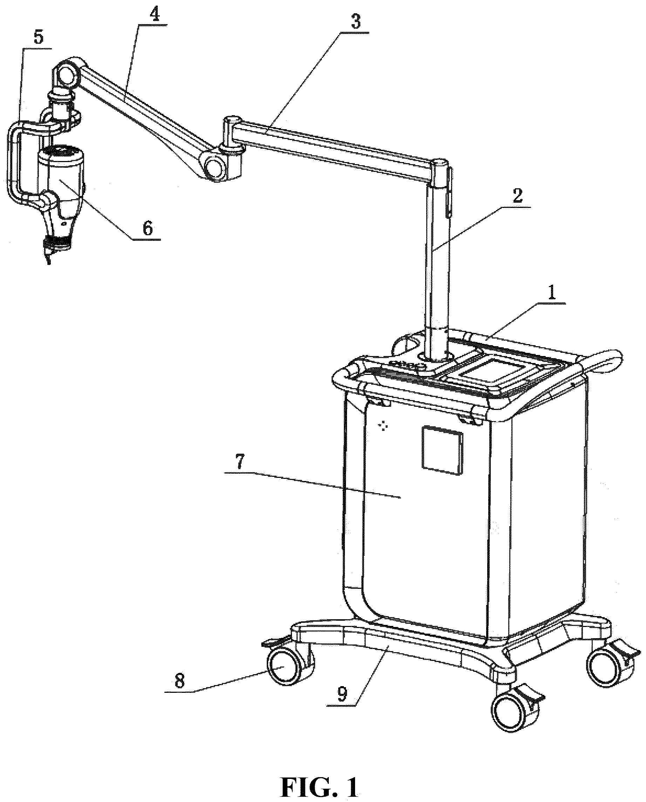

FIG. 1 is a schematic structural diagram according to the present invention.
FIG. 2 is a schematic circuit diagram according to the present invention.

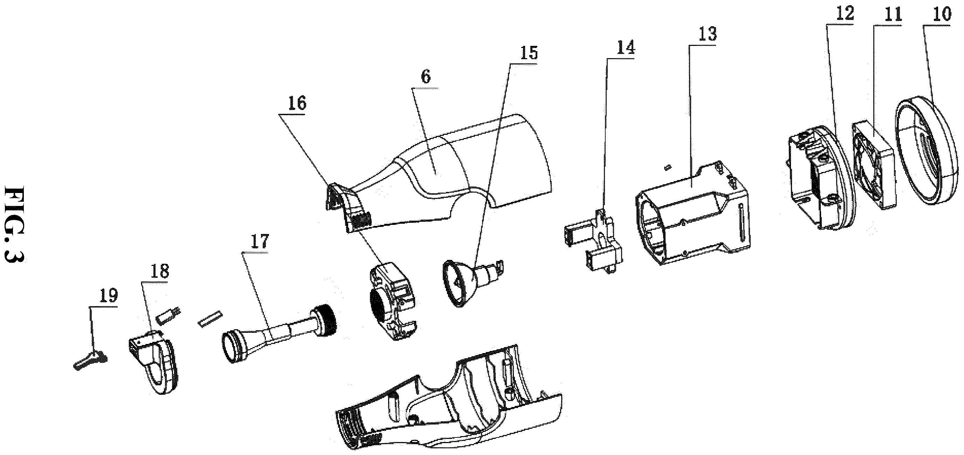

FIG. 3 is an exploded view of a treatment head according to the present invention.

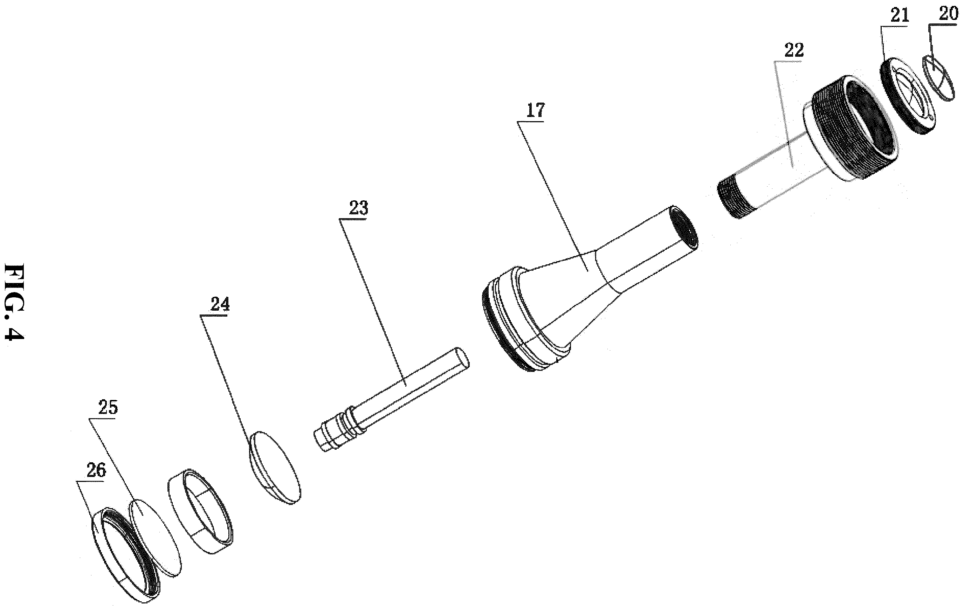

FIG. 4 is an exploded view of an optical tube according to the present invention.

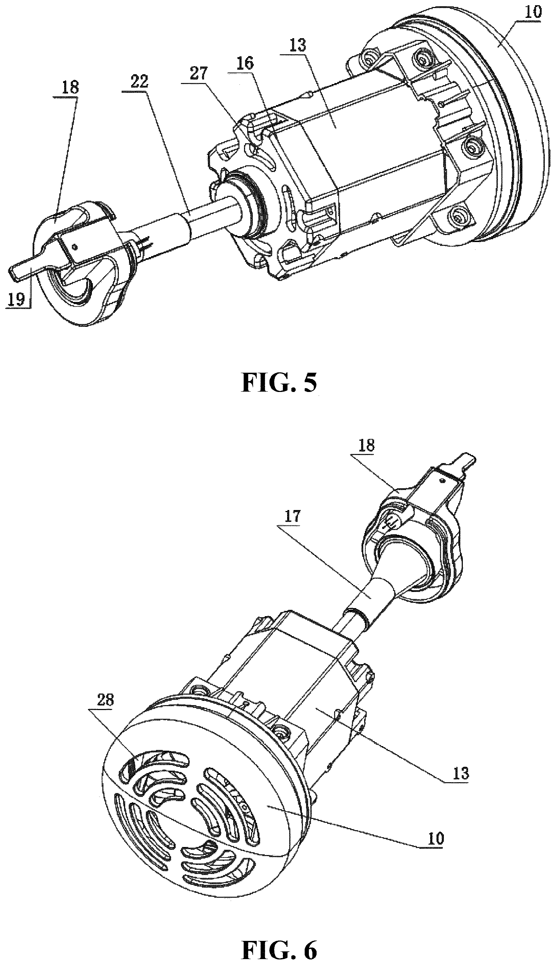

FIG. 5 is a schematic circuit diagram of the treatment head with a housing removed according to the present invention.
FIG. 6 is another perspective view of the treatment head with the housing removed according to the present invention.

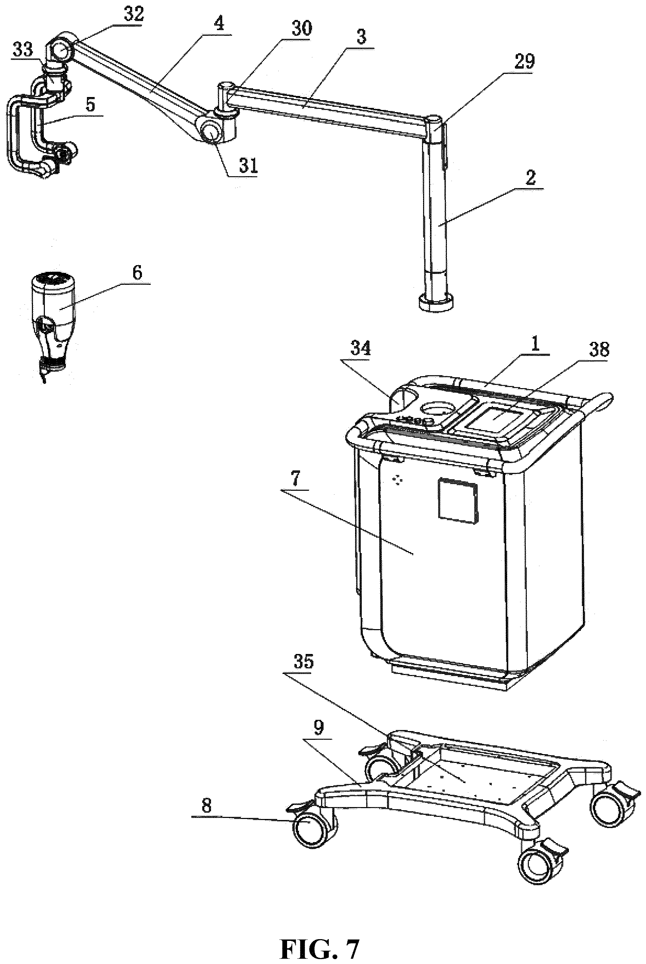

FIG. 7 is an exploded view according to the present invention.

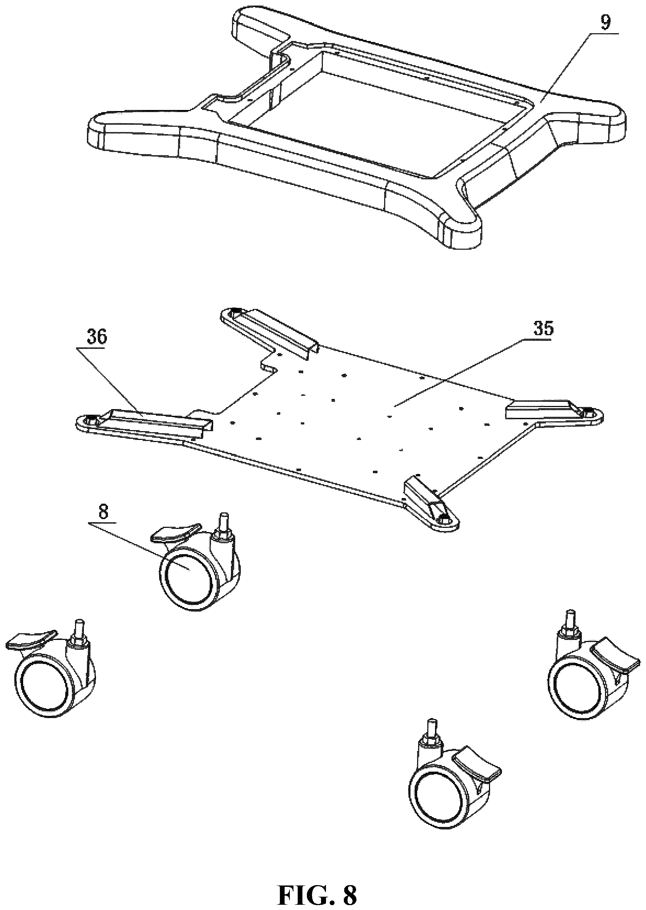

FIG. 8 is an exploded view of a base according to the present invention.

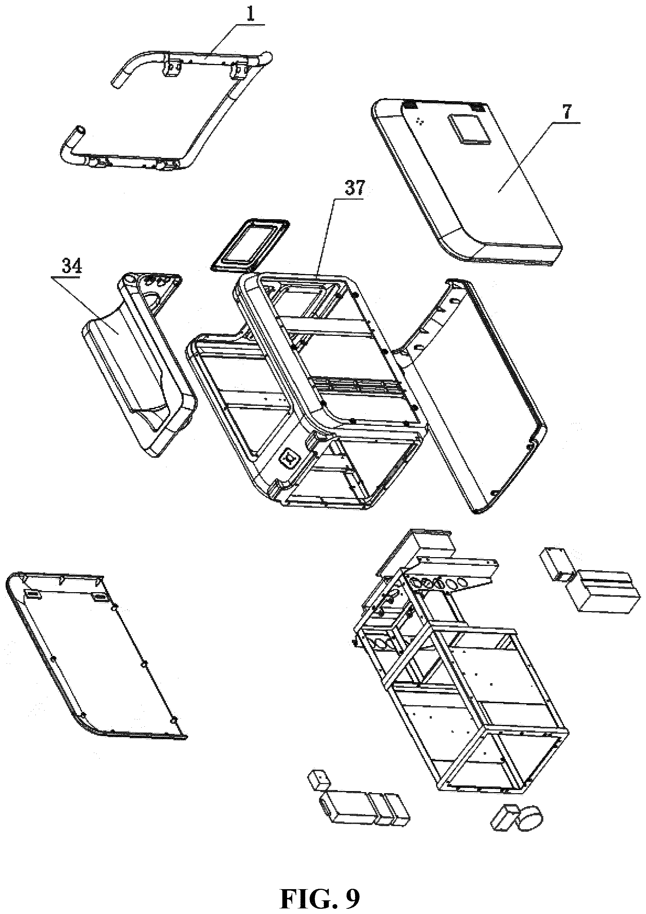

FIG. 9 is an exploded view of a carrier body according to the present invention.

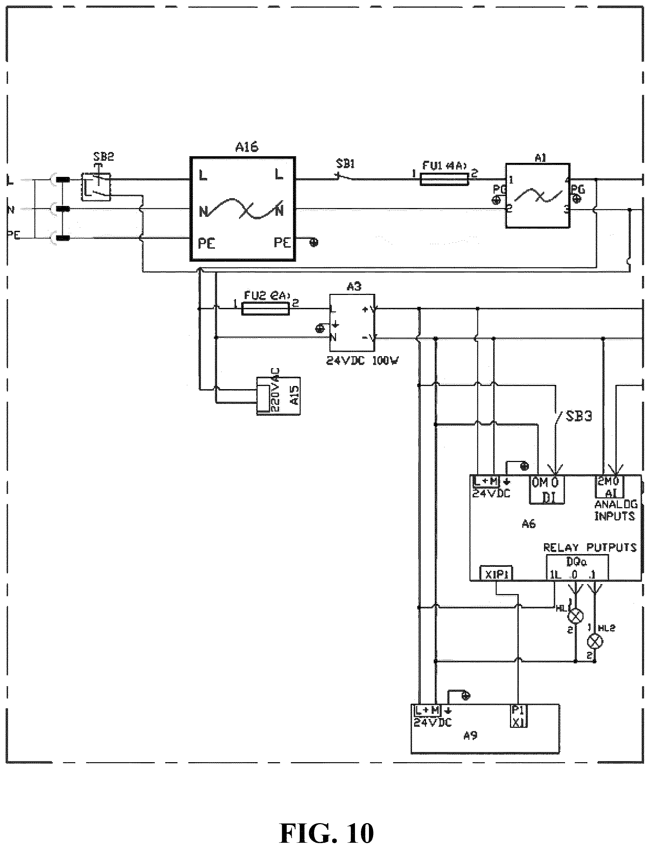

FIG. 10 is an enlarged view of part A in FIG. 2.

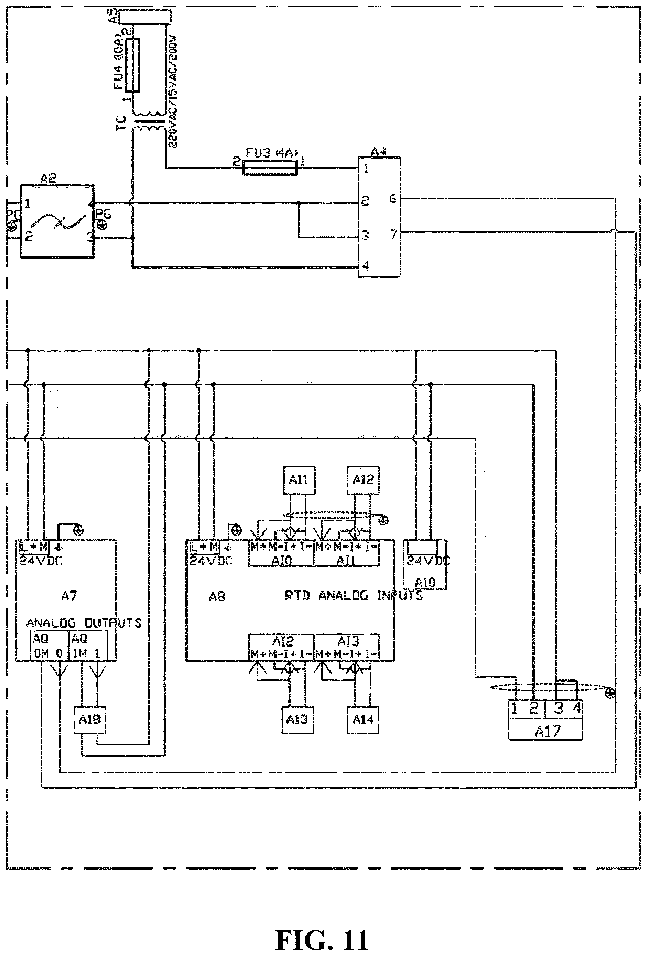

FIG. 11 is an enlarged view of part B in FIG. 2.

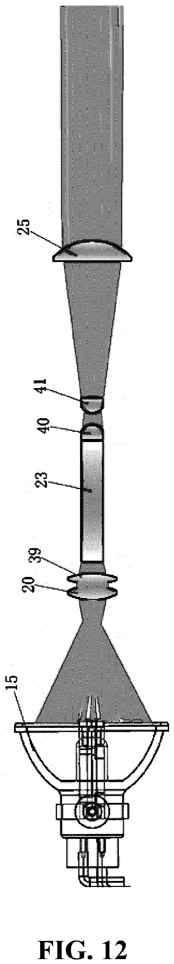

FIG. 12 is a schematic diagram of a light path of an optical system according to the present invention.
FIG. 13 is a control flow chart according to the present invention.

In the drawings, 1 is an armrest, 2 is an upright column, 3 is a horizontal rocker arm, 4 is a connecting rocker arm, 5 is a handle, 6 is a treatment head housing, 7 is a carrier body housing, 8 is a wheel, 9 is a base plate cover, 10 is a rear end protective cover, 11 is a treatment head interior heat dissipation fan, 12 is a light extinction barrel mounting base, 13 is a rear light extinction barrel, 14 is a reflector cup mounting base, 15 is a reflector cup, 16 is a front light extinction barrel, 17 is a front half, 18 is a limiting component, 19 is a limiting silicone head, 20 is a front convex lens, 21 is a lens fixing member, 22 is a rear half, 23 is an integrator rod, 24 is a convex lens, 25 is a lens, 26 is a lens fixing member, 27 is an arc-shaped heat dissipation hole, 28 is a strip-shaped heat dissipation hole, 29 is a first vertical rotating shaft, 30 is a second vertical rotating shaft, 31 is a first horizontal rotating shaft, 32 is a second horizontal rotating shaft, 33 is a third vertical rotating shaft, 34 is a treatment head receiving groove, 35 is a base plate, 36 is a U-shaped reinforcement profile, 37 is a structural frame, 38 is a touch screen, 39 is a rear convex lens, 40 is a front convex lens, and 41 is a rear convex lens.

### Detailed Description of Embodiments

As shown in the drawings, the present invention comprises a infrared light generating device, and a bearing and driving part configured to bear the infrared light generating device and capable of driving the infrared light generating device to be disposed at a suitable position. The infrared light generating device comprises an optical system and a light source part. The light source part is connected to a light source control circuit.

The optical system comprises a light source focusing group, a light homogenizing group, a homogenized-light focusing and focus regulating group, and a homogenized-light collimating group arranged in sequence from front to back. The light source part comprises a reflector cup 15 and a light source provided at the center of the reflector cup 15. The reflector cup 15 is provided on the front side of the light source focusing group. The coordinated use of the light source focusing group, the light homogenizing group, the homogenized-light focusing and focus regulating group, the homogenized-light collimating group, and the reflector cup 15 can improve the utilization rate of the light source, satisfy the spot size and the parallelism of light columns, and improve the immune induction therapy effect.

The light source focusing group uses two convex lenses, or uses one convex lens, or uses two plano-convex lenses and one biconvex lens, or uses a nonstandard spherical lens.

The light homogenizing group uses an integrator rod 23. The integrator rod 23 distributes the incoming light uniformly.

The integrator rod 23 has a diameter of 7 mm and a length of 40 mm.

The integrator rod 23 is a cylindrical glass rod with both ends coated with antireflection films and an outer side wall coated with an internal reflection film. The coating of the antireflection films and the internal reflection film facilitates band screening.

A front convex lens 20 in the two convex lenses of the light source focusing group has a front surface radius of 18 mm, a second surface radius of -17 mm, and a center thickness of 4.95 mm; and a rear convex lens 39 in the two convex lenses of the light source focusing group has a front surface radius of 24 mm, a second surface radius of -24 mm, and a center thickness of 3.25 mm. The front convex lens 20 focuses the light emitted by the light source part into light with a certain angle. The rear convex lens 39 focuses the divergent light with a certain angle after being focused by the front convex lens 20 into the light homogenizing group behind.

The homogenized-light focusing and focus regulating group uses two convex lenses, or uses one convex lens 24.

A front convex lens 40 in the two convex lenses of the homogenized-light focusing and focus regulating group has a front surface radius of 150.96 mm, a second surface radius of -4.17 mm, and a center thickness of 4.98 mm; and a rear convex lens 41 in the two convex lenses of the homogenized-light focusing and focus regulating group has a front surface radius of 150.96 mm, a second surface radius of -4.17 mm, and a center thickness of 4.98 mm. The front convex lens 40 focuses the uniform divergent light obtained after entering the integrator rod 23, and the rear convex lens 41 regulates the focal length of the focused uniform divergent light.

The homogenized-light collimating group uses a lens 25, and the lens 25 has a rear surface radius of 17.5 mm. The homogenized-light collimating group makes the incoming uniform divergent light into parallel light.

The lens 25 may be made of a calcium fluoride material, which transmits infrared light widely, satisfies the requirement of a wide band, and ensures that the following light band contains light of different wavelengths.

The distance from the rear end of the reflector cup 15 to the front convex lens of the light source focusing group is 41.14 mm, the distance between the front convex lens of the light source focusing group and the rear convex lens of the light source focusing group is 0.25 mm, the distance from the rear convex lens of the light source focusing group to the integrator rod 23 is 4.29 mm, the distance from the light homogenizing group to the front convex lens of the homogenized-light focusing and focus regulating group is 0.47 mm, the distance between the front convex lens of the homogenized-light focusing and focus regulating group and the rear convex lens of the homogenized-light focusing and focus regulating group is 4 mm, and the distance between the rear convex lens of the homogenized-light focusing and focus regulating group and the homogenized-light collimating group is 45.29 mm.

The reflector cup 15 uses a nonlinear secondary reflector cup. The inner surface of the reflector cup 15 is coated with a gold reflecting film, wherein the band is 200 nm-2500 nm, and the reflectivity is greater than 99.8%. The reflector 15 may use a high-boron glass reflector cup.

The light source uses a halogen tungsten lamp.

The infrared light generating device comprises a treatment head housing 6, and the front end of the treatment head housing 6 is a light outlet hole. A light extinction barrel mounting base 12, a rear light extinction barrel 13, a front light extinction barrel 16, and an optical tube are arranged in the treatment head housing 6 in sequence from back to front. The light source part is provided in the rear of the rear light extinction barrel 13, and the optical system is provided in the optical tube.

The bearing and driving part comprises a base, a carrier body, and a rocker arm assembly. The carrier body is provided on the base. One end of the rocker arm assembly is connected to the upper end of the carrier body, and the other end of the rocker arm assembly is connected to the treatment head housing 6. The light source control circuit is provided in the carrier body.

The light source control circuit comprises a main control part, a lesion temperature detection part, a power supply part, and an output regulation part. A detection signal input port of the main control part is connected to a detection signal output port of the lesion temperature detection part, and a control signal output port of the main control part is connected to a control signal input port of the output regulation part. An electric energy output port of the power supply part is connected to an electric energy input port of the main control part, an electric energy input port of the lesion temperature detection part, and an electric energy input port of the output regulation part, respectively.

The present invention further comprises a treatment head temperature detection part and a carrier body temperature detection part. The detection signal input port of the main control part is connected to a detection signal output port of the treatment head temperature detection part and a detection signal output port of the carrier body temperature detection part, respectively.

The present invention further comprises a system operation state indication part, a system alarm state indication part, and a human-computer interaction part. The control signal output port of the main control part is connected to a control signal input port of the system operation state indication part and a control signal input port of the system alarm state indication part, respectively. A signal transmission port of the main control part is connected to a signal transmission port of the human-computer interaction part. An electric energy input port of the human-computer interaction part is connected to the electric energy output port of the power supply part.

The present invention further comprises a treatment head heat dissipation part and a carrier body heat dissipation part. An electric energy input port of the treatment head heat dissipation part and an electric energy input port of the carrier body heat dissipation part are respectively connected to the electric energy output port of the power supply part.

The output regulation part uses a solid-state voltage regulator.

The lesion temperature detection part uses an infrared temperature sensor. The infrared temperature sensor is a non-contact sensor and does not directly contact the lesion, thereby greatly reducing the possibility of contamination of devices.

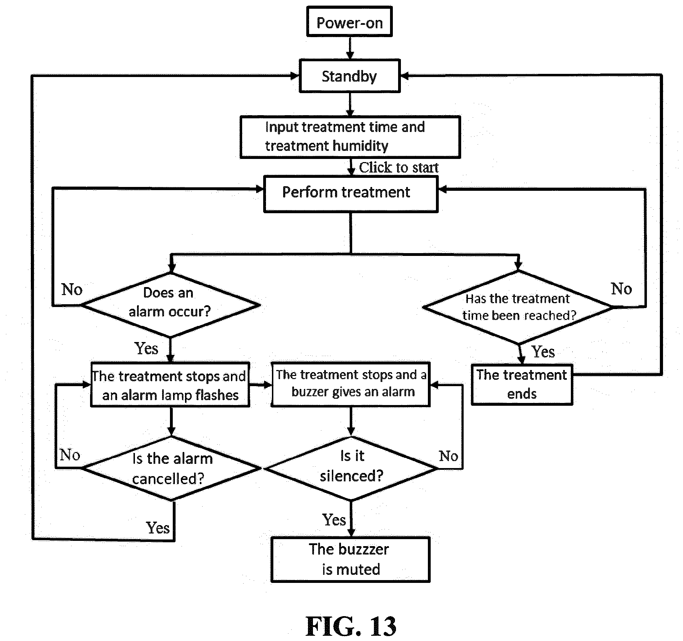

As shown in FIG. 13, standby: the fans of the treatment head and the carrier body continue to run, and the controller gives a 0 V voltage to the solid-state voltage regulator.

Alarm and alarm release: an alarm is activated when the temperature detected by the treatment head temperature detection part is higher than 80 degrees Celsius, and the alarm is canceled when the temperature is lower than 40 degrees Celsius; an alarm is activated when the temperature detected by the carrier body temperature detection part is higher than 50 degrees Celsius, and the alarm is canceled when the temperature is lower than 40 degrees Celsius; and an alarm is activated when the temperature detected by the lesion temperature detection part is higher than 50 degrees Celsius, and the alarm is canceled when the temperature is lower than 50 degrees Celsius.

Silencing: when an alarm occurs, there will be an alarm sound, and when a silencer button is pressed, the alarm sound will no longer arise until the next alarm occurs.

The power supply part comprises a pulse train suppressor, a first filter, a second filter, and a direct-current power source; an input port of the pulse train suppressor is a mains access port; an output port of the pulse train suppressor is connected to an input port of the second filter, an input port of the direct-current power source, and the electric energy input port of the carrier body heat dissipation part, respectively, through the first filter; an output port of the second filter is connected to the electric energy input port of the output regulation part; and an output port of the direct-current power source is connected to the electric energy input port of the main control part, the electric energy input port of the lesion temperature detection part, the electric energy input port of the human-computer interaction part, and the electric energy input port of the treatment head heat dissipation part, respectively.

The pulse train suppressor uses a DTS4221-3NE-W20-D16 pulse train suppressor A16. The first filter and the second filter use RSHN-2006L filters A1 and A2. The direct-current power source uses an MSP-100-24 direct-current power source A3.An input L port of A16 is connected to a wiring terminal of an SB2 output end of a GQ22-11ZE220V switch; the other wiring terminal of the SB2 output end is respectively connected to pin 2 of A2, pin 3 of A1, an N port of A3, and an electric energy input N port of the carrier body heat dissipation part; an SB2 input end is a mains L wiring terminal; an input N port of A16 is a mains N wiring terminal; and an input PE port of A16 is a mains PE wiring terminal. An output L port of A16 is connected to pin 1 of A1 through a switch SB1 and a ceramic fuse FU1 in sequence; an output N port of A16 is connected to pin 2 of A1; pin 4 of A1 is connected to pin 1 of A2, one end of a ceramic fuse FU2, and an electric energy input L port of the carrier body heat dissipation part, respectively; and the other end of the ceramic fuse FU2 is connected to port L of A3.

The switch SB1 may use an inclination switch. When the inclination angle of device is greater than 15 degrees, the device is powered off.

The main control part comprises a PLC controller, an analog output module, and an analog input module; a detection signal input port of the PLC controller is connected to the detection signal output port of the lesion temperature detection part, and a signal transmission port of the PLC controller is connected to the signal transmission port of the human-computer interaction part; the analog output module is connected to the control signal input port of the output regulation part and the control signal input port of the system alarm state indication part, respectively; and the analog input module is connected to the detection signal output port of the treatment head temperature detection part and the detection signal output port of the carrier body temperature detection part, respectively.

The PLC controller uses a 6ES7214-1HG40-0XB0 controller A6; the analog output module uses a 6ES7232-4HB32-0XB0 module A7; the analog input module uses a 6ES7231-5PD32-0XB0 module A8; port L+ of A6, port L+ of A7, and port L+ of A8 are all connected to port +V of the MSP-100-24 direct-current power source A3; and port M of A6, port M of A7, and port M of A8 are all connected to port -V of A3.Port 0 of a DI part of A6 is connected to port +V of A3 through a GQ16F-10 silencer button SB3, port 0M of the DI part of A6 is connected to the port - V of A3, and port 2M of an AI part of A6 is connected to the port -V of A3.Port 1L of a DQa part of A6 is connected to the port +V of A3.

PLC communication control can control treatment temperature, and temperature rise curve and duration.

The carrier body heat dissipation part uses a carrier body interior heat dissipation fan, and the treatment head heat dissipation part uses a treatment head interior heat dissipation fan 11.

The infrared temperature sensor uses an MLX90614-ESF-BCF sensor A17; pin 1 of A17 is connected to port 0 of the AI part of A6, pin 2 of A17 is connected to port -V of A3, and pins 3 and 4 of A17 are connected to the port +V of A3.

The MI,X90614-ESF-BCF non-contact infrared temperature sensor provides accurate readings within an error range of plus or minus 0.5 degrees Celsius without touching the lesions.

The human-computer interaction part uses a touch screen 38.

The touch screen 38 uses a 6AV2124-0GC01-0AX0 touch screen A9; port L+ of A9 is connected to the port +V of A3, port M of A9 is connected to the port -V of A3, and port X1P1 of A9 is connected to port X1P1 of A6.

The system operation state indication part uses an indicator lamp HL1; the system alarm state indication part comprises an indicator lamp HL2 and a buzzer A18; port 0 of a DQa part of A6 is connected to the port -V of A3through HL1, and port 1 of the DQa part of A6 is connected to the port -V of A3 through HL2; a control signal input port of A18 is connected to port 1M and port 1 of A7, and a power port of A18 is respectively connected to the ports +V and -V of A3.

The solid-state voltage regulator uses an LTVDH-220V-15A solid-state voltage regulator A4; pin 1 of A4 is connected to one end of a TC primary side of a 220VAC/15VAC/200W transformer through a ceramic fuse FU3; the other end of the TC primary side is connected to pin 3 of A2 and pin 4 of A4, respectively; pin 4 of A2 is connected to pins 2 and 3 of A4, respectively; one end of a TC secondary side is connected to one end of a power terminal of the light source through a ceramic fuse FU4; the other end of the power terminal of the light source is connected to the other end of the TC secondary side; and pin 7 of A4 is connected to port 0M of A7, and pin 6 of A4 is connected to port 0 of A7. The LTVDH-220V-15A solid-state voltage regulator performs 0-220VAC voltage regulation output through an analog voltage signal. The internal circuit is designed in isolation and is thus safer.

The transformer may be 220 VAC input and 15 VAC output isolation type ring transformer.

The treatment head temperature detection part comprises two thermistors; one end of one of the thermistors is respectively connected to port M+ and port I+ of an AI0 part of A8, and the other end is respectively connected to port M- and port I- of the AI0 part of A8; one end of the other thermistor is respectively connected to port M+ and port I+ of an AI1 part of A8, and the other end is respectively connected to port M- and port I- of the AI1 part of A8.

The carrier body temperature detection part comprises two thermistors; one end of one of the thermistors is respectively connected to port M+ and port I+ of an AI2 part of A8, and the other end is respectively connected to port M- and port I- of the AI2 part of A8; one end of the other thermistor is respectively connected to port M+ and port I+ of an AI3 part of A8, and the other end is respectively connected to port M- and port I- of the AI3 part of A8.

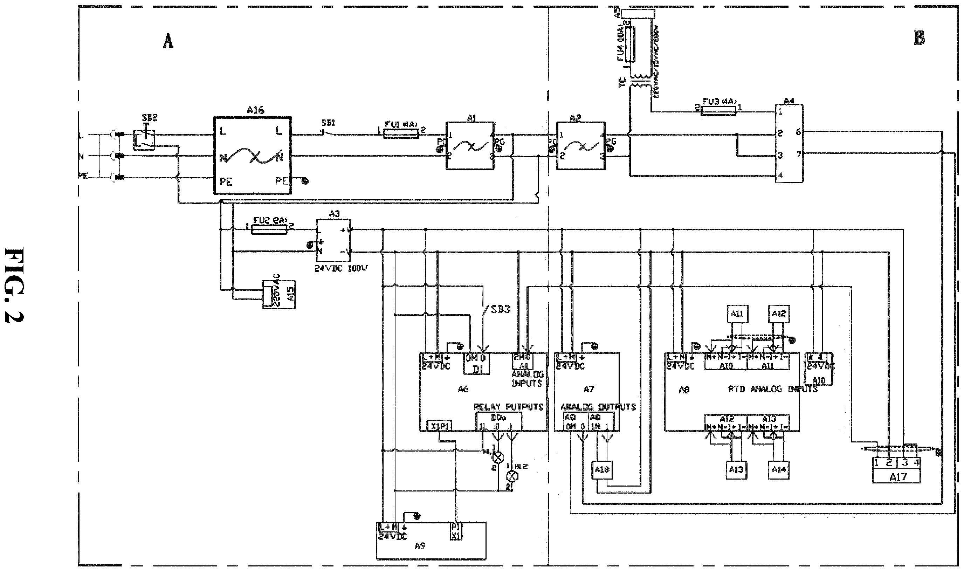

As shown FIG. 2, from the silencer button SB3 to a DI interface 0 channel of the controller A6, a pulse on-off instruction of the silencer button SB3 is received. From a treatment temperature sensor A17 to an AI interface 0 channel of the controller, the lesion temperature detected by the sensor is received. Through 4-20ma signal transmission, the interference of parallel lines thereto is reduced, and the element size of the head part is reduced. From a DQa interface 0 channel of the controller to a yellow indicator lamp HL1, it is configured to indicate a system operating state. From a DQa interface 1 channel of the controller to a blue indicator lamp HL2, it is configured to indicate a system alarm state. From an AQ interface 0 channel of a controller analog output expansion module to a signal receiving interface of the solid-state voltage regulator A4, it is configured to regulate the outlet voltage of the solid-state voltage regulator. From an AQ interface 1 channel of the controller analog output expansion module to a signal receiving interface of a buzzer A18, it is configured to make a system alarm sound.

From a thermistor A11 to an AIO channel of a controller analog input expansion module, and from a thermistor A12 to an AI1 channel of the controller analog input expansion module, it is configured to detect the temperature of the treatment head. Component damage and patient burns due to excessively high internal temperature of the treatment head are prevented.

From a thermistor A13 to an AI2 channel of the controller analog input expansion module, and from a thermistor A14 to an AI3 channel of the controller analog input expansion module, it is configured to detect the temperature of the carrier body. Component damage due to excessively high internal temperature of the carrier body is prevented. When detecting temperature by collecting resistance values of the thermistors, there is no transmission link, so that the measurement accuracy is improved, and the thermistors have a relatively long service life.

The treatment head housing 6 comprises a shell with portions on two sides snap-fitted together and a rear end protective cover 10. The protective cover is threadedly connected to the shell. The protective cover is provided with strip-shaped heat dissipation holes 28. The use of threaded connection can prevent a patient from easily removing the protective cover, resulting in injury and damage.

The rear end of the light extinction barrel mounting base 12 is provided with a treatment head interior heat dissipation fan 11.

The rear light extinction barrel 13 and the front light extinction barrel 16 both use aluminum alloy light extinction barrels, and the inner walls are subjected to extinction processing; the front end of the front light extinction barrel 16 is provided with an internal threaded hole connected to the rear end of the optical tube, and the front light extinction barrel 16 around the internal thread hole is provided with arc-shaped heat dissipation holes 27; a reflector cup mounting base 14 is provided in the rear light extinction barrel 13, and the reflector cup mounting base 14 comprises a mounting hole in the rear end and support arms on both sides; and the front ends of the support arms are bent inward, and the end faces are in an arc shape corresponding to the front arc-shaped surface of the reflector cup 15. Aluminum alloy has better heat resistance, and in addition, the inner walls are subjected to extinction processing, so that the light is more directional and the effect of directional projection is improved. The structure of the reflector cup mounting base 14 enables the direction of light to be straight and avoids shaking of the reflector cup 15.

The optical tube comprises a front half 17 and a rear half 22; the front half 17 is threadedly connected to the rear half 22; the rear end of the rear half 22 is provided with an external thread connected to the front light extinction barrel 16; both the rear end of the rear half 22 and the front end of the rear half 17 are provided with lens fixing members 21 and 26; and the lens fixing members 21 and 26 are threadedly connected to the optical tube; the middle of the optical tube is provided with a light homogenizing group mounting member.

The rear end of the rear half 22 is provided with an external thread connected to the front light extinction barrel 16, so that the distance between the optical tube and the reflector cup 15 can be adjusted, and thus the device is more precise and accurate. At a distance of 55 mm from a light outlet, the spot diameter can be regulated to be less than Φ25 mm.

The lens fixing member uses a threaded structure to press the lens to a desired position to fix it.

The shell uses an ABS engineering plastic shell, and the front side wall of the shell is provided with strip-shaped heat dissipation holes 28.

It is stated that a infrared light generating device, the total duration of a single light exposure is 1,740 seconds, heating is completed to present temperature within 60 seconds, maintaining the preset temperature is adjusted in 61-240 seconds, the cooling is in 241-420 seconds, reduced to less than the preset temperature 1.5 degrees Celsius and adjusted, at the same time satisfied tan45 degrees (Angle of temperature rise curve), the heating is in 421-600 seconds, increased to more than the preset temperature 1.5 degree Celsius and adjusted, at the same time satisfied tan45 degrees, the cooling is in 601-660 seconds, reduced to the preset temperature, the cooling is in 661-840 seconds, reduced to less than the preset temperature 1.8 degree Celsius and adjusted, at the same time satisfied tan45 degrees, the heating is in 841-1020 seconds, increased to more than preset temperature 1.8 degree Celsius and adjusted, at the same time satisfied tan45 degrees, the cooling is in 1021-1080 seconds, reduced to preset temperature, the cooling is in 1081-1200 seconds, reduced to less than preset temperature 1.8 degree Celsius and adjusted, at the same time satisfied tan45 degrees, the heating is in 1021-1320 seconds, increased to more than preset temperature 1.8 degrees Celsius and adjusted, at the same time satisfied tan45 degrees, the cooling is in 1321-1380 seconds, reduced to less than preset temperature, the cooling is in 1381-1500 seconds, reduced to less than the preset temperature 1.8 degree Celsius and adjusted, at the same time satisfied tan45 degrees, the heating is in 1501-1620 seconds, increased to more than preset temperature 1.8 degrees Celsius and adjusted, at the same time satisfied tan45 degrees, the cooling is in 1621-1740 seconds, reduced to preset temperature.

It is stated that when the preset temperature is maintained in 61-240 seconds: the peak value of infrared light wave with 60% of the maintenance power in 61-75 seconds is 0.8-1.2um, and the preset temperature is adjusted in 76-240 seconds.

It is stated that the heating is in 421-600 seconds: the peak value of infrared light wave with 54% of the maintenance power in 421-435 seconds is 1.5-1.7um, and the heating is in 436-600 seconds.

It is stated that the cooling is in 601-660 seconds: the peak value of infrared light wave with 38% of the maintenance power in 601-615 seconds is 2.5-2.7um, and the cooling is in 616-660 seconds.

It is stated that the cooling is in 661-840 seconds: the peak value of infrared light wave with 42% of maintenance power in 661-675 seconds is 2.2-2.6um, and the cooling is in 676-840 seconds.

It is stated that the heating is in 841-1020 seconds: the peak value of infrared light wave with 30% of maintenance power in 841-855 seconds is 2.8-3.0um, and the heating is in 856-1020 seconds.

It is stated that the cooling is in 1021-1080 seconds: the peak value of infrared light wave with 56% of maintenance power in 1021-1035 seconds is 1.7-1.9um, and the cooling is in 1036-1080 seconds.

It is stated that the cooling is in 1081-1200 seconds: the peak value of infrared light wave with 60% of maintenance power in 1081-1095 seconds is 0.8-1.2um, and the cooling is in 1096-1200 seconds.

It is stated that the heating is in 1021-1320 seconds: the peak value of infrared light wave with 40% of maintenance power in 1021-1035 seconds is 2.6-2.8um, and the cooling is in 1036-1320 seconds.

It is stated that the cooling is in 1321-1380 seconds: the peak value of infrared light wave with 47% of maintenance power in 1321-1335 seconds is 1.8-2.1um, and the cooling is in 1336-1380 seconds.

It is stated that the cooling is in 1381-1500 seconds: the peak value of infrared light wave with 60% of maintenance power in 1381-1395 seconds is 0.8-1.2um and the cooling is in 1395~1500 seconds.

It is stated that the heating is in 1501-1620 seconds: the peak value of infrared light wave with 30% of maintenance power in 1501-1515 seconds is 2.8-3.0um and the heating is in 1516-1620 seconds.

It is stated that the cooling is in 1621-1740 seconds: the peak value of infrared light wave with 54% of maintenance power in 1621-1635 seconds is 1.5-1.7um and the cooling is down to present temperature in 1636-1740 seconds

It is states that a infrared light generating device, the total duration of a single light exposure is 1,740 seconds, heating is completed to present temperature within 60 seconds, the peak value of infrared light wave with 56% of the maintenance power in 61-75 seconds is 1.7-1.9um, keeping present temperature is adjusted in 76-240 seconds, the peak value of infrared light wave with 40% of the maintenance power in 256-420 seconds is 2.6-2.8um. and the temperature is reduced in 256-420 seconds, reduced to less than the present temperature 1.5 degrees Celsius and adjusted at the same time satisfied tan45 degrees, the peak value of infrared light wave with 54% of the maintenance power in 421-435 seconds is 1.5-1.7um, the heating is in 436-600 seconds, increased to more than preset temperature 1.5 degrees Celsius and adjusted, at the same time satisfied tan45 degrees, the peak value of infrared light wave with 30% of the maintenance power in 601-615 seconds is 2.8-3.0um, the cooling is in 616-660 seconds, reduced to preset temperature, the peak value of infrared light wave with 42% of the maintenance power in 661-675 seconds is 2.2-2.6um, the cooling is in 676-840 seconds, reduced to less than preset temperature 1.8 degrees Celsius and adjusted, at the same time satisfied tan45 degrees, the peak value of infrared light wave with 38% of the maintenance power in 841-855 seconds is 2.5-2.7um, the heating is in 856-1020 seconds, increased to more than preset temperature 1.8 degrees Celsius and adjusted, at the same time satisfied tan45 degrees, the peak value of infrared light wave with 60% of the maintenance power in 1021-1035 seconds is 0.8-1.2um, the cooling is in 1036-1080 seconds, reduced to preset temperature, the peak value of infrared light wave with 60% of the maintenance power in 1081-1095 seconds is 0.8-1.2um, the cooling is in 1096-1200 seconds, reduced to less than preset temperature 1.8 degrees Celsius and adjusted, at the same time satisfied tan45 degrees, the peak value of infrared light wave with 40% of the maintenance power in 1021-1035 seconds is 2.6-2.8um, the heating is in 1036-1320 seconds, increased to more than 1.8 degrees Celsius and adjusted, at the same time satisfied tan45 degrees, the peak value of infrared light wave with 42% of the maintenance power in 1321-1335 seconds is 2.2-2.6um, the cooling is in 1336-1380 seconds, reduced to preset temperature, the peak value of infrared light wave with 60% of the maintenance power in 1381-1395 seconds is 0.8-1.2um, the cooling is in 1395-1500 seconds, reduced to less than preset temperature 1.8 degrees Celsius and adjusted, at the same time satisfied tan40 degrees, the peak value of infrared light wave with 30% of the maintenance power in 1501-1515 seconds is 2.8-3.0um, the heating is in 1516-1620 seconds, increased to more than 1.8 degrees Celsius and adjusted, at the same time satisfied tan45 degrees, the peak value of infrared light wave with 54% of the maintenance power in 1621-1635 seconds is 1.5-1.7um, the cooling is in 1636-1740 seconds, reduced to preset temperature.

It is stated that a infrared light generating device, the total duration of a single light exposure is 1,740 seconds, heating is completed to present temperature within 60 seconds, the peak value of infrared light wave with 42% of the maintenance power in 61-75 seconds is 2.2-2.6um, keeping present temperature is adjusted in 76-240 seconds, the peak value of infrared light wave with 30% of the maintenance power in 241-255 seconds is 2.8-3.0um, the cooling is in 256-420 seconds, reduced to less than preset temperature 1.5 degrees Celsius and adjusted, at the same time satisfied tan45 degrees, the peak value of infrared light wave with 54% of the maintenance power in 421-435 seconds is 1.5-1.7um, the heating is in 436-600 seconds, increased to more than preset temperature 1.5 degrees Celsius and adjusted, at the same time satisfied tan45 degrees, the peak value of infrared light wave with 60% of the maintenance power in 601-615 seconds is 0.8-1.2um, the cooling is in 616-660 seconds, reduced to preset temperature, the peak value of infrared light wave with 42% of the maintenance power in 661-675 seconds is 2.2-2.6um, the cooling is in 676-840 seconds, reduced to less than preset temperature 1.8 degrees Celsius and adjusted and at the same time satisfied tan45 degrees, the peak value of infrared light wave with 38% of the maintenance power in 841-855 seconds is 2.5-2.7um, the heating is in 856-1020 seconds, increased to more than preset temperature 1.8 degrees Celsius and adjusted, at the same time satisfied tan45 degrees, the peak value of infrared light wave with 47% of the maintenance power in 1021-1035 seconds is 1.8-2.1um, the cooling is in 1036-1080 seconds, reduced to preset temperature, the peak value of infrared light wave with 60% of the maintenance power in 1081-1095 seconds is 0.8-1.2um, the cooling is in 1096-1200 seconds, reduced to less than preset temperature 1.8 degrees Celsius and adjusted, at the same time satisfied tan45 degrees, the peak value of infrared light wave with 40% of the maintenance power in 1021-1035 seconds is 2.6-2.8um, the heating is in 1036-1320 seconds, increased to more than preset temperature 1.8 degrees Celsius and adjusted, at the same time satisfied tan45 degrees, the peak value of infrared light wave with 54% of the maintenance power in 1321-1335 seconds is 1.5-1.7um, the cooling is in 1336-1380 seconds, reduced to preset temperature, the peak value of infrared light wave with 60% of the maintenance power in 1381-1395 seconds is 0.8-1.2um, the cooling is in 1395-1500 seconds, reduced to less than preset temperature 1.8 degrees Celsius and adjusted, at the same time satisfied tan45 degrees, the peak value of infrared light wave with 60% of the maintenance power in 1501-1515 seconds is 0.8-1.2um, the heating is in 1516-1620 seconds, increased to more than preset temperature 1.8 degrees Celsius and adjusted, at the same time satisfied tan45 degrees, the peak value of infrared light wave with 60% of the maintenance power in 1621-1635 seconds is 0.8-1.2um, the cooling is in 1636-1740 seconds, reduced to preset temperature.

The above control methods can significantly improve the therapeutic effect.

The present temperature is 41, 42, 43 or 44°C.

The front end of the treatment head housing 6 is provided with a limiting component 18, the limiting component 18 has a limiting silicone head 19 protruding forward, and the lesion temperature detection part is provided on the limiting component 18.

The limiting silicone head 19 can ensure that the limiting component 18 keeps a certain distance from the lesion during the treatment process.

The limiting silicone head 19 has a length of 5 mm to 10 mm.

The treatment head temperature detection part is provided in the treatment head housing 6.

The base comprises a base plate 35; a base plate cover 9 is snapped onto the base plate 35; the middle of the base plate cover 9 is provided with a carrier body mounting groove, and the lower ends of the four corners of the base plate 35 are provided with wheels 8.

The wheels 8 use 10-inch universal wheels.

The base plate 35 uses an iron base plate, the upper ends of the four corners of the base plate 35 are welded with U-shaped reinforcement profiles 36, and the base plate 35 is provided with carrier body connecting holes. Because the U-shaped reinforcement profiles 36 are welded, the structure is hard and not easy to deform.

The base plate cover 9 uses an ABS engineering plastic base plate cover.

The carrier body may be connected to the base plate 35 by using screws.

The carrier body comprises a structural frame 37; the carrier body housing 7 is provided around the structural frame 37; the upper end of the carrier body housing 7 is provided with an armrest 1 and the human-computer interaction part; the lower end of the structural frame 37 is connected to the base plate 35; and the light source control circuit is provided in the structural frame 37.

The armrest 1 is a U-shaped armrest surrounding two lateral sides and the rear of the carrier body, and the rear end of the armrest 1 is tilted upward. The U-shaped armrest surrounds three sides, thereby making the movement under push more convenient and easier.

The front carrier body housing 7 of the carrier body is an L-shaped structure; the vertical surface of the front end of the front carrier body housing 7 is provided with a vertical treatment head receiving groove 34, and the horizontal surface of the rear upper end of the front carrier body housing 7 is connected to the front upper end of the structural frame 37; both sides of the front end of the armrest 1 are bent inward, and the bend of the front carrier body housing 7 is provided with connecting insertion holes corresponding to the bent portions on the front end of the armrest 1. Because the front surface is designed to have a recess, when the therapy apparatus is stored, the treatment head can be placed in the recess to save space, and in addition, the function of protecting the treatment head can be also achieved.

The carrier body housing 7 is connected to the structural frame 37 through an inserting structure and screws, thereby facilitating disassembly and assembly during maintenance.

The carrier body housing 7 uses an ABS engineering plastic housing.

The structural frame 37 uses a metal structural frame, and thus is firm in structure.

Pre-installation holes for the parts, housings and base may be reserved on the structural frame 37.

The rocker arm assembly comprises an upright column 2, a horizontal rocker arm 3, a connecting rocker arm 4, and a handle 5; the lower end of the upright column 2 is connected to the carrier body, and the upper end of the upright column 2 is connected to the rear end of the horizontal rocker arm 3 through a first vertical rotating shaft 29; the front end of the horizontal rocker arm 3 is connected to the rear end of a horizontal connecting block through a second vertical rotating shaft 30; the front end of the horizontal connecting block is connected to the rear end of the connecting rocker arm 4 through a first horizontal rotating shaft 31; the front end of the connecting rocker arm 4 is connected to the upper end of a vertical connecting block through a second horizontal rotating shaft 32; the lower end of the vertical connecting block is connected to the upper end of the handle 5 through a third vertical rotating shaft 33; and the lower end of the handle 5 is connected to the treatment head housing 6.

The upright column 2 increases the height of a treatable interval for the therapy apparatus.

The upright column 2 may also be configured as a liftable structure, and each rotating shaft may also be configured as an electric rotating shaft.

The lifting ranges of the horizontal rocker arm 3 and the connecting rocker arm 4 can be designed to 500 MM, plus or minus 60 degrees of supine angle, and greater than 270 degrees of X-axis rotation, thereby achieving full coverage of a sitting body.

The lower end of the handle 5 is connected to the treatment head housing 6 through a wire-passing damping shaft.

The handle 5 is a horizontal double U-shaped handle 5; the upper ends of U-shaped handles 5 on both sides are bent toward each other and connected to the lower end of the vertical connecting block through the third vertical rotating shaft 33, and the lower ends of the U-shaped handles 5 on both sides are bent toward each other and connected to both sides of the treatment head housing 6.

The lower ends of the U-shaped handles 5 on both sides are bent toward each other and connected to both sides of the treatment head housing 6 through the wire-passing damping shaft. The wire-passing damping shaft can enable the Y-axis rotation of the treatment head to be more than 270 degrees.

The design of the U-shaped handles 5 on both sides provides to good comfort, and enables medical staff to more conveniently adjust the degrees of freedom of the therapy apparatus.

The use process of the present invention is described below in conjunction with the drawings.

The process comprises: holding the handle 5 in hand, pulling the treatment head to a suitable position so that the treatment light spot emitted by the treatment head can directly toward the lesions and at an appropriate distance; and manually setting the irradiation temperature (such as 45 degrees Celsius, the temperature setting range may be 40°C to 46°C) and the irradiation duration (such as 30 minutes) by means of the touch screen 38, and pressing a start button; or selecting an automatic program and performing treatment according to the program set in the system.

## Claims

1. An immunity induction therapy apparatus, comprising a infrared light generating device, and a bearing and driving part configured to bear the infrared light generating device and capable of driving the infrared light generating device to be disposed at a suitable position, wherein the infrared light generating device comprises a light source part and an optical system configured to regulate and output light emitted by the light source part, and the light source part is connected to a light source control circuit wherein the optical system comprises a light source focusing group, a light homogenizing group, a homogenized-light focusing and focus regulating group, and a homogenized-light collimating group arranged in sequence from front to back; the light source part comprises a reflector cup (15) and a light source provided at the center of the reflector cup; and the reflector cup is provided on the front side of the light source focusing group and the light homogenizing group comprises an integrator rod (23) .

2. The immunity induction therapy apparatus according to claim 1, wherein the integrator rod (23) is a cylindrical glass rod with both ends coated with antireflection films and an outer side wall coated with an internal reflection film.

3. The immunity induction therapy apparatus according to claim 1 or 2, wherein the light source focusing group comprises two convex lenses (20, 39).,

4. The immunity induction therapy apparatus according to claim 1, 2 or 3, wherein the homogenized-light focusing and focus regulating group, comprises two convex lenses (40,41)

5. The immunity induction therapy apparatus according to any of the preceding claims claims, wherein the infrared light generating device comprises a treatment head housing (6), and a front end of the treatment head housing is a light outlet hole; a light extinction barrel mounting base (12), a rear light extinction barrel (13), a front light extinction barrel (16), and an optical tube are arranged in the treatment head housing (6) in sequence from back to front; and the light source part is provided in the rear of the rear light extinction barrel (13), and the optical system is provided in the optical tube.

6. The immunity induction therapy apparatus according to claim 5, wherein the bearing and driving part comprises a base, a carrier body, and a rocker arm assembly; the carrier body is provided on the base; one end of the rocker arm assembly is connected to an upper end of the carrier body, and the other end of the rocker arm assembly is connected to the treatment head housing (6); and the light source control circuit is provided in the carrier body.

7. The immunity induction therapy apparatus according to claim 6, wherein the light source control circuit comprises a main control part, a lesion temperature detection part, a power supply part, and an output regulation part; a detection signal input port of the main control part is connected to a detection signal output port of the lesion temperature detection part, and a control signal output port of the main control part is connected to a control signal input port of the output regulation part; and an electric energy output port of the power supply part is connected to an electric energy input port of the main control part, an electric energy input port of the lesion temperature detection part, and an electric energy input port of the output regulation part, respectively.

8. The immunity induction therapy apparatus according to claim 7, further comprising a treatment head temperature detection part and a carrier body temperature detection part, the detection signal input port of the main control part being connected to a detection signal output port of the treatment head temperature detection part and a detection signal output port of the carrier body temperature detection part, respectively.

9. The immunity induction therapy apparatus according to claim 7, further comprising a system operation state indication part, a system alarm state indication part, and a human-computer interaction part, the control signal output port of the main control part being connected to a control signal input port of the system operation state indication part and a control signal input port of the system alarm state indication part, respectively; a signal transmission port of the main control part being connected to a signal transmission port of the human-computer interaction part; and an electric energy input port of the human-computer interaction part being connected to the electric energy output port of the power supply part.

10. The immunity induction therapy apparatus according to claim 7, further comprising a treatment head heat dissipation part and a carrier body heat dissipation part, an electric energy input port of the treatment head heat dissipation part and an electric energy input port of the carrier body heat dissipation part being respectively connected to the electric energy output port of the power supply part.

## Patentansprüche

1. Immuninduktionstherapievorrichtung, umfassend ein Infrarotlichterzeugungsgerät, und ein Lager- und Antriebsteil zum Lagern des Infrarotlichterzeugungsgeräts konfiguriert und zum Antrieb des Infrarotlichterzeugungsgeräts fähig, um in eine geeignete Stelle angeordnet zu werden, wobei das Infrarotlichterzeugungsgerät ein Lichtquelleteil und ein optisches System umfasst, das zum Regeln und zur Ausgabe des vom Lichtquelleteil abgestrahlten Lichtes konfiguriert ist, und das Lichtquelleteil mit einer Lichtquellesteuerschaltung verbunden ist, wobei das optische System eine Lichtquellefokussierungsgruppe, eine Lichthomogenisierungsgruppe, eine Homogenisiertes-Lichtfokussierungs- und Fokusregelgruppe, und eine Homogenisiertes-Lichtkollimiergruppe umfasst, die der Reihe nach von vorne nach hinten angeordnet sind; das Lichtquelleteil eine Reflektorschale (15) und eine Lichtquelle umfasst, die im Zentrum der Reflektorschale vorgesehen ist; und die Reflektorschale auf der Vorderseite der Lichtquellefokussierungsgruppe vorgesehen ist, und die Lichthomogenisierungsgruppe einen Integratorstab (23) umfasst

2. Immuninduktionstherapievorrichtung nach Anspruch 1, wobei der Integratorstab (23) ein zylindrischer Glasstab ist, dessen beiden Enden mit Antireflexionsfolien beschichtet sind, und dessen einer Außenseitenwand mit einer internen Reflexionsfolie beschichtet ist.

3. Immuninduktionstherapievorrichtung nach Anspruch 1 oder 2, wobei die Lichtquellefokussierungsgruppe zwei konvexe Linsen (20,39) umfasst.

4. Immuninduktionstherapievorrichtung nach Anspruch 1, 2 oder 3, wobei die Homogenisiertes-Lichtfokussierungs- und Fokusregelgruppe zwei konvexe Linsen (40,41) umfasst.

5. Immuninduktionstherapievorrichtung nach einer der vorhergehenden Ansprüchen, wobei das Infrarotlichterzeugungsgerät ein Behandlungskopfgehäuse (6) umfasst, und ein Vorderende des Behandlungskopfgehäuses eine Lichtauslassöffnung ist; ein Lichtabschaltungszylindermontagesockel (12), ein Hinterlichtabschaltungszylinder (13), ein Vorderlichtabschaltungszylinder (16), und ein optisches Rohr in dem Behandlungskopfgehäuse der Reihe nach von vorne nach hinten angeordnet sind; und das Lichtquelleteil im hinteren Teil des Hinterlichtabschaltungszylinders (13) vorgesehen ist, und das optische System im optischen Rohr vorgesehen ist.

6. Immuninduktionstherapievorrichtung nach Anspruch 5, wobei das Lager- und Antriebsteil einen Sockel, einen Trägerkörper und ein Kipphebelgestell umfasst; der Trägerkörper auf dem Sockel vorgesehen ist; eine Ende des Kipphebelgestells mit dem Oberende des Trägerkörpers verbunden ist, und das andere Ende des Kipphebelgestells mit dem Behandlungskopfgehäuse (6) verbunden ist; und die Lichtquellesteuerschaltung im Trägerkörper vorgesehen ist.

7. Immuninduktionstherapievorrichtung nach Anspruch 6, wobei die Lichtquellesteuerschaltung ein Hauptsteuerteil, ein Läsionstemperaturerfassungsteil, ein Stromversorgungsteil und ein Ausgaberegelteil umfasst; ein Erfassungssignaleingangsanschluss des Hauptsteuerteils mit einem Erfassungssignalausgangsanschluss des Läsionstemperaturerfassungsteils verbunden ist, und ein Steuersignalausgangsanschluss des Hauptsteuerteils mit einem Steuersignaleingangsanschluss des Ausgaberegelteils verbunden ist; und ein Elektroenergieausgangsanschluss des Stromversorgungsteils jeweils mit einem Elektroenergieeingangsanschluss des Hauptsteuerteils, einem Elektroenergieeingangsanschluss des Läsionstemperaturerfassungsteils und einem Elektroenergieeingangsanschluss des Ausgaberegelteils verbunden ist.

8. Immuninduktionstherapievorrichtung nach Anspruch 7, wobei sie weiter ein Behandlungskopftemperaturerfassungsteil und ein Trägerkörpertemperaturerfassungsteil umfasst, wobei der Erfassungssignaleingangsanschluss des Hauptsteuerteils jeweils mit einem Erfassungssignalausgangsanschluss des Behandlungskopftemperaturerfassungsteil und einem Erfassungssignalausgangsanschluss des Trägerkörpertemperaturerfassungsteils verbunden ist.

9. Immuninduktionstherapievorrichtung nach Anspruch 7, wobei sie weiter ein Systembetriebszustandsanzeigeteil, ein Systemalarmzustandsanzeigeteil, ein Mensch-Computer-Interaktionsteil, wobei der Steuersignalausgangsanschluss des Hauptsteuerteils jeweils mit einem Steuersignaleingangsanschluss des Systembetriebszustandsanzeigeteils und einem Steuersignaleingangsanschluss des Systemalarmzustandsanzeigeteils verbunden ist; wobei ein Signalübertragungsanschluss des Hauptsteuerteils mit einem Signalübertragungsanschluss des Mensch-Computer-Interaktionsteils verbunden ist; und ein Elektroenergieeingangsanschluss des Mensch-Computer-Interaktionsteils mit dem Elektroenergieausgangsanschluss des Stromversorgungsteils verbunden ist.

10. Immuninduktionstherapievorrichtung nach Anspruch 7, wobei sie weiter ein Behandlungskopfwärmeabfuhrteil und ein Trägerkörperwärmeabfuhrteil umfasst, wobei ein Elektroenergieeingangsanschluss des Behandlungskopfwärmeabfuhrteils und ein Elektroenergieeingangsanschluss des Trägerkörperwärmeabfuhrteils jeweils mit dem Elektroenergieausgangsanschluss des Stromversorgungsteils verbunden sind.

## Revendications

1. Appareil thérapeutique à induction immunitaire comprenant un dispositif générateur de lumière infrarouge, et une partie d'amenée transporteuse configurée pour transporter le dispositif générateur de lumière infrarouge, et capable d'amener le dispositif générateur de lumière infrarouge à être disposé dans une position appropriée, dans lequel le dispositif générateur de lumière infrarouge comprend une partie formant source de lumière et un système optique configuré pour réguler et produire la lumière émise par la partie formant source de lumière, et la partie formant source de lumière est connectée à un circuit de réglage de la source de lumière, où le système optique comprend un groupe de focalisation de lumière, un groupe d'homogénéisation de lumière, un groupe de focalisation de lumière homogénéisée et de régulation de focus, et un groupe de collimation de lumière homogénéisée disposés de l'avant vers l'arrière ; la partie formant source de lumière comprenant un réflecteur en forme de coupelle (15) et une source de lumière prévue au centre du réflecteur en forme de coupelle ; et le réflecteur en forme de coupelle est prévu sur le côté frontal du groupe de focalisation de source de lumière et le groupe d'homogénéisation de lumière comprend une barre formant intégrateur (23).

2. Appareil thérapeutique à induction immunitaire selon la revendication 1, dans lequel la barre formant intégrateur (23) est une barre en verre cylindrique dont les deux extrémités sont revêtues de films anti-réfléchissants et dont une paroi latérale extérieure est revêtue d'un film de réflexion interne.

3. Appareil thérapeutique à induction immunitaire selon la revendication 1 ou 2, dans lequel le groupe focalisation de source de lumière comprend deux lentilles convexes (20, 39).

4. Appareil thérapeutique à induction immunitaire selon la revendication 1, 2 ou 3, dans lequel le groupe de focalisation de lumière homogénéisée et de régulation de focus comprend deux lentilles convexes (40,41).

5. Appareil thérapeutique à induction immunitaire selon l'une quelconque des revendications précédentes, dans lequel le dispositif générateur de lumière infrarouge comprend un boîtier de tête de traitement (6), et une extrémité frontale du boîtier de tête de traitement est une cavité émettrice de lumière; une embase de montage de cylindre d'extinction de lumière (12), un cylindre d'extinction de lumière arrière (13), un cylindre d'extinction de lumière frontal (16), et un tube optique sont disposés dans le boîtier de tête de traitement (6) en séquence de l'arrière vers l'avant ; et la partie formant source de lumière est prévue à l'arrière du cylindre d'extinction de lumière arrière (13), et le système optique est prévu dans le tube optique.

6. Appareil thérapeutique à induction immunitaire selon la revendication 5, dans lequel la pièce d'amenée transporteuse comprend une embase , un corps transporteur, un assemblage formant bras culbuteur, le corps transporteur est prévu sur l'embase ; une extrémité de l'assemblage formant bras culbuteur est raccordée à l'extrémité supérieure du corps transporteur, et l'autre extrémité de l'assemblage formant bras culbuteur et raccordée au boîtier de tête de traitement (6) ; et le circuit de réglage de la source lumineuse est prévu dans le corps transporteur.

7. Appareil thérapeutique à induction immunitaire selon la revendication 6, dans lequel le circuit de réglage de la source de lumière comprend une partie principale de commande, une partie de détection de température de lésion, une partie d'alimentation en énergie électrique, et une partie de régulation de sortie ; une porte d'entrée de signal de détection de la partie principale de commande est connectée à une porte de sortie de signal de détection de la partie de détection de température de lésion, et une porte de sortie de signal de commande de la partie principale de commande est connectée à la porte d'entrée de signal de commande de la partie de régulation de sortie ; et une porte de sortie d'énergie électrique de la partie d'alimentation en énergie électrique est connectée respectivement à la porte d'entrée de la partie principale de commande, à une porte d'entrée d'énergie électrique de la partie de détection de température de lésion, et à une porte d'entrée d'énergie électrique de la partie de régulation de sortie.

8. Appareil thérapeutique à induction immunitaire selon la revendication 7, comprenant une partie de détection de température de tête de traitement et une partie de détection de température de corps transporteur, la porte d'entrée de signal de détection de la partie principale de commande étant connectée respectivement à une porte de sortie de signal de détection de la partie de détection de température de tête de traitement et à une porte de sortie de signal de détection de la partie de détection de température de corps transporteur.

9. Appareil thérapeutique à induction immunitaire selon la revendication 7, comprenant en outre une partie indicatrice de l'état opérationnel du système, une partie indicatrice de l'état d'alarme du système, une partie interactive entre l'homme et l'ordinateur, la porte de sortie de signal de commande de la partie principale de commande étant connectée respectivement à la porte d'entrée de signal de commande de la partie indicatrice de l'état opérationnel du système et à la porte d'entrée de signal de commande de la partie indicatrice de l'état d'alarme du système ; une porte de transmission de signal de la partie principale de commande étant connectée à une porte de transmission de signal de partie interactive entre l'homme et l'ordinateur ; une porte d'entrée de signal d'énergie électrique de la partie interactive entre l'homme et l'ordinateur étant connectée à la porte de sortie d'énergie électrique de la partie d'alimentation en énergie électrique.

10. Appareil thérapeutique à induction immunitaire selon la revendication 7, comprenant en outre une partie de dissipation thermique de tête de traitement et une partie de dissipation thermique de corps de transporteur, une porte d'entrée d'énergie électrique de la partie de dissipation thermique de tête de traitement et une porte d'entrée d'énergie électrique de la partie de dissipation thermique de corps de transporteur étant connectées respectivement à la porte de sortie d'énergie électrique de la partie d'alimentation en énergie électrique.
